# EUROPÄISCHE PATENTSCHRIFT

(11) **EP 2 569 277 B1**
(45) Veröffentlichungstag und Bekanntmachung des Hinweises auf die Patenterteilung: **22.07.2015**
(21) Anmeldenummer: 11720740.7
(22) Anmeldetag: 09.05.2011
(51) Int. Cl.: C07C 229/38, A61K 31/197, C07D 263/22, C07D 265/32, C07D 213/30, A61K 31/4015, A61K 31/4245

(54) **SUBSTITUIERTE 8-ALKOXY-2-AMINOTETRALIN-DERIVATE UND IHRE VERWENDUNG**
SUBSTITUTED 8-ALKOXY-2-AMINOTETRALIN DERIVATIVES, AND USE THEREOF
DÉRIVÉS DE 8-ALCOXY-2-AMINOTÉTRALINE SUBSTITUÉS ET LEUR UTILISATION

(30) Priorität: 14.05.2010 DE 102010020553
(43) Veröffentlichungstag der Anmeldung: 20.03.2013
(73) Patentinhaber: Bayer Intellectual Property GmbH, 40789 Monheim (DE)
(72) Erfinder: HÜBSCH, Walter, 42113 Wuppertal (DE); HAHN, Michael, 40764 Langenfeld (DE); VAKALOPOULOS, Alexandros, 40721 Hilden (DE); LI, Volkhart, Min-Jian, 42553 Velbert (DE); WUNDER, Frank, 42117 Wuppertal (DE); STASCH, Johannes-Peter, 42651 Solingen (DE); SCHLEMMER, Karl-Heinz, 42113 Wuppertal (DE); STOLL, Friederike, 40215 Düsseldorf (DE); LINDNER, Niels, 42115 Wuppertal (DE)
(86) Internationale Anmeldenummer: PCT/EP2011/057390
(87) Internationale Veröffentlichungsnummer: WO 2011/141409

(56) Entgegenhaltungen:
- WO-A1-02/070462

## Beschreibung

Die vorliegende Anmeldung betrifft neue, substituierte 8-Alkoxy-2-aminotetralin-Derivate, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise [O.V. Evgenov et al., Nature Rev. Drug Disc. 5 (2006), 755].

In den letzten Jahren wurden Substanzen identifiziert, die die lösliche Guanylatcyclase direkt, d.h. ohne vorherige Freisetzung von NO stimulieren. Mit dem Indazolderivat YC-1 wurde erstmals ein NO-unabhängiger, jedoch Häm-abhängiger Stimulator der sGC beschrieben [Evgenov et al., *ibid.*]. Ausgehend von YC-1 wurden weitere Substanzen gefunden, welche eine höhere Potenz als YC-1 besitzen und keine relevante Hemmung von Phosphodiesterasen (PDE) aufweisen. Dies führte zur Identifizierung der Pyrazolopyridin-Derivate BAY 41-2272, BAY 41-8543 und BAY 63-2521. Diese Verbindungen bilden gemeinsam mit den kürzlich publizierten, strukturell diversen Substanzen CMF-1571 und A-350619 die neue Klasse der sGC-Stimulatoren [Evgenov et al., *ibid.*]. Gemeinsames Charakteristikum dieser Substanzklasse ist eine NO-unabhängige und selektive Aktivierung der hämhaltigen sGC. Darüber hinaus zeigen die sGC-Stimulatoren in Kombination mit NO einen synergistischen Effekt auf die sGC-Aktivierung, welcher auf einer Stabilisierung des Nitrosyl-Häm-Komplexes basiert. Die genaue Bindungsstelle der sGC-Stimulatoren an der sGC ist bis heute Gegenstand der Diskussion. Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten Stimulatoren stimulierbar [Evgenov et al., *ibid.*].

Darüber hinaus wurden NO- und Häm-unabhängige sGC-Aktivatoren, mit BAY 58-2667 als Prototyp dieser Klasse, identifiziert. Gemeinsame Charakteristika dieser Substanzen sind, dass sie in Kombination mit NO nur einen additiven Effekt auf die Enzymaktivierung ausüben, und dass die Aktivierung des oxidierten oder hämfreien Enzyms im Vergleich zum hämhaltigen Enzym deutlich stärker ist [Evgenov et al., *ibid.*; J.P. Stasch et al., Br. J. Pharmacol. 136 (2002), 773; J.P. Stasch et al., J. Clip. Invest. 116 (2006), 2552]. Spektroskopische Untersuchungen lassen erkennen, dass BAY 58-2667 die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Propionsäuren der Hämgruppe als auch für die Wirkung von BAY 58-2667 zwingend erforderlich ist. Vor diesem Hintergrund wird angenommen, dass die Bindungsstelle von BAY 58-2667 an der sGC identisch zur Bindungsstelle der Hämgruppe ist [J.P. Stasch et al., J. Clin. Invest. 116 (2006), 2552].

Aufgabe der vorliegenden Erfindung war die Bereitstellung neuer Verbindungen, welche in der oben beschriebenen Weise als NO- und Häm-unabhängige Aktivatoren der löslichen Guanylatcyclase Wirken und als solche zur Behandlung und zur Prävention insbesondere kardiovaskularer Erkrankungen eingesetzt werden können.

Verschiedene Aminodicarbonsäure-Derivate zur Behandlung von Herz-Kreislauf-Erkrankungen wurden zuvor in WO 01/19780-A2, WO 02/070459-A1, WO 02/070460-A1, WO 02/070461-A1, WO 02/070462-A1 und WO 02/070510-A2 beschrieben. Insbesondere für ZNS-Erkrankungen therapeutisch einsetzbare 2-Aminotetralin-Derivate sind aus EP 0 041 488-A1, EP 0 064 964-A1, EP 0 270 947-A2, EP 0 272 534-A2, WO 90/15047-A1, FR 2 659 853-A1, WO 99/62505-A2 und WO 2005/012291-A1 bekannt.

Gegenstand der vorliegenden Erfindung sind nunmehr Verbindungen der allgemeinen Formel (I) 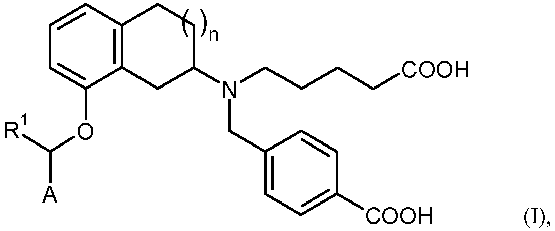 in welcher
- n: 7für die Zahl 1 steht,
- R¹: für Wasserstoff oder Methyl steht,
und
- A: für eine Gruppe der Formel 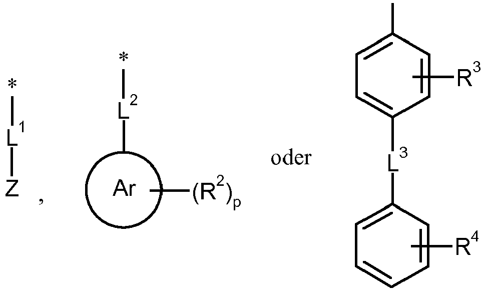 steht, worin
* die jeweilige Verknüpfungsstelle mit dem Rest des Moleküls kennzeichnet,
L¹ geradkettiges (C₁-C₅)-Alkandiyl, das ein- oder zweifach mit Methyl und ein- oder zweifach mit Fluor substituiert sein kann, bedeutet,
Z Wasserstoff, Fluor, Cyano, Trifluormethyl oder eine Gruppe der Formel 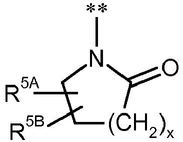 bedeutet, worin
** die Verknüpfungsstelle mit der Gruppe L¹ kennzeichnet,
x die Zahl 1, 2 oder 3 darstellt, wobei eine dieser CH₂-Gruppen gegen -O-ausgetauscht sein kann,
und
R^{5A} und R^{5B} unabhängig voneinander Wasserstoff oder Methyl darstellen,
L² eine Bindung oder geradkettiges (C₁-C₅)-Alkandiyl bedeutet,
Ar Phenyl oder 5- oder 6-gliedriges Heteroaryl mit bis zu drei Ring-Heteroatomen aus der Reihe N, O und/oder S bedeutet,
R² einen Substituenten ausgewählt aus der Reihe Fluor, Chlor, Brom, Cyano, (C₁-C₄)-Alkyl, Trifluormethyl, (C₁-C₄)-Alkoxy und Trifluormethoxy bedeutet,
p die Zahl 0, 1 oder 2 bedeutet,
wobei im Falle, dass der Substituent R² zweifach auftritt, seine individuellen Bedeutungen gleich oder verschieden sein können,
L³ eine Bindung, -O-, -CH₂-, -CH₂-CH₂- oder -CH=CH- bedeutet, und
R³ und R⁴ unabhängig voneinander Wasserstoff oder einen Substituenten ausgewählt aus der Reihe Fluor, Chlor, Brom, Cyano, (C₁-C₄)-Alkyl, Trifluormethyl, (C₁-C₄)-Alkoxy und Trifluormethoxy bedeuten,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze üblicher Mineralsäuren, Carbonsäuren und Sulfonsäuren, wie z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Trisethanolamin, Dicyclohexylamin, Dimethylaminoethanol, Prokain, Dibenzylamin, *N*-Methylpiperidin, *N*-Methylmorpholin, Lysin, Arginin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Der Begriff "Prodrugs" bezeichnet Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₄-Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, *n*-Propyl, Isopropyl, *n*-butyl, Isobutyl, *sec*.-Butyl und *tert*.-Butyl.
(C₁-C₅)-Alkandiyl und (C₂-C₄-Alkandiyl stehen im Rahmen der Erfindung für einen geradkettigen, α,ω-divalenten Alkylrest mit 1 bis 5 bzw. 2 bis 4 Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen) und Pentan-1,5-diyl (1,5-Pentylen).
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n-*Propoxy, Isopropoxy, *n*-Butoxy, Isobutoxy, *sec*.-Butoxy und *tert*.-Butoxy.
5- oder 6-gliedriges Heteroaryl steht im Rahmen der Erfindung für einen aromatischen Heterocyclus (Heteroaromaten) mit insgesamt 5 bzw. 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, 1,2-Oxazolyl (Isoxazolyl), 1,3-Oxazolyl, 1,2-Thiazolyl (Isothiazolyl), 1,3-Thiazolyl, 1,2,3-Triazolyl, 1,2,4-Triazolyl, 1,2,4-Oxadiazolyl, 1,3,4-Oxadiazolyl, 1,2,4-Thiadiazolyl, 1,3,4-Thiadiazolyl, Pyridyl, Pyrimidinyl, Pyridazinyl, Pyrazinyl, 1,2,4-Triazinyl und 1,3,5-Triazinyl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

Bevorzugt im Rahmen der vorliegenden Erfindung sind Verbindungen der Formel (I), in welcher
- n: für die Zahl 1 steht,
- R¹: für Wasserstoff steht,
und
- A: für eine Gruppe der Formel 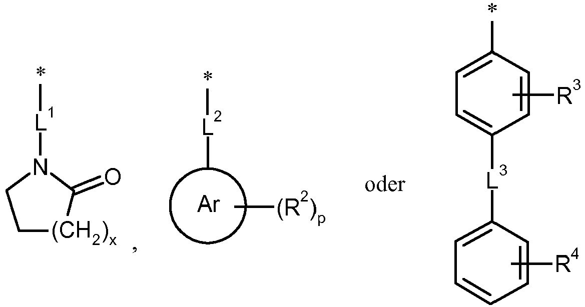 steht, worin
* die jeweilige Verknüpfungsstelle mit dem Rest des Moleküls kennzeichnet,
L¹ geradkettiges (C₂-C₄)-Alkandiyl bedeutet,
x die Zahl 1 oder 2 bedeutet, wobei eine dieser CH₂-Gruppen gegen -O- ausgetauscht sein kann,
L² eine Bindung oder -CH₂- bedeutet,
Ar Phenyl, Pyridyl, 1,2,4-Oxadiazolyl oder 1,3,4-Oxadiazolyl bedeutet,
R² einen Substituenten ausgewählt aus der Reihe Fluor, Chlor, (C₁-C₄)-Alkyl und Trifluormethyl bedeutet,
p die Zahl 0 oder 1 bedeutet,
L³ eine Bindung oder -CH₂-CH₂- bedeutet, und
R³ und R⁴ unabhängig voneinander Wasserstoff oder einen Substituenten ausgewählt aus der Reihe Fluor, Chlor, (C₁-C₄)-Alkyl und Trifluormethyl bedeuten, sowie ihre Salze, Solvate und Solvate der Salze.

Besonders bevorzugt im Rahmen der vorliegenden Erfindung sind Verbindungen der Formel (I), in welcher
- n: für die Zahl 1 steht,
- R¹: für Wasserstoff steht,
und
- A: für eine Gruppe der Formel 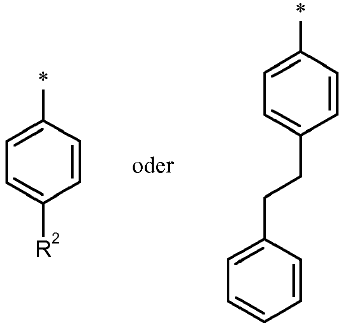
steht, worin
- *: die jeweilige Verknüpfungsstelle mit dem Rest des Moleküls kennzeichnet
und
- R²: Methyl, Ethyl, Isopropyl oder *tert*.-Butyl bedeutet,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Verbindung der Formel (II) 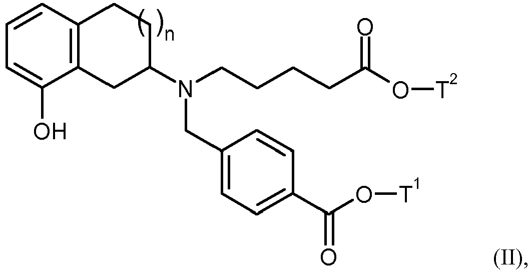 in welcher n die oben angegebene Bedeutung hat
und
T¹ und T² gleich oder verschieden sind und für (C₁-C₄)-Alkyl stehen,
in Gegenwart einer Base mit einer Verbindung der Formel (III) 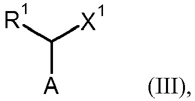 in welcher R¹ und A die oben angegebenen Bedeutungen haben
und
- X¹: für eine Abgangsgruppe wie beispielsweise Chlor, Brom, Iod, Mesylat, Triflat oder Tosylat steht,
zu einer Verbindung der Formel (IV) 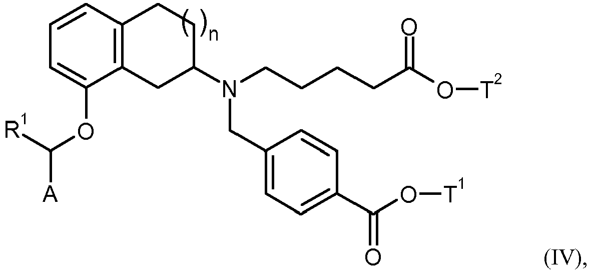 in welcher n, R¹, A, T¹ und T² jeweils die oben angegebenen Bedeutungen haben, umsetzt und diese dann durch Hydrolyse der Ester-Gruppierungen -C(O)OT¹ und -C(O)OT² in die entsprechende Dicarbonsäure der Formel (I) überführt
und die so erhaltenen Verbindungen der Formel (I) gegebenenfalls in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

Als inerte Lösungsmittel für den Verfahrensschritt (II) + (III) → (IV) eignen sich beispielsweise Ether wie Diethylether, Diisopropylether, Methyl-*tert*.-butylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, oder dipolar-aprotische Lösungsmittel wie Aceton, Methylethylketon, Acetonitril, *N*,*N*-Dimethylformamid (DMF), *N*,*N*-Dimethylacetamid (DMA), Dimethylsulfoxid (DMSO), *N*,*N*'-Dimethylpropylenharnstoff (DMPU) oder *N*-Methylpyrrolidinon (NMP). Ebenso ist es möglich, Gemische solcher Lösungsmittel einzusetzen. Bevorzugt wird Acetonitril oder Dimethylformamid verwendet.

Für den Verfahrensschritt (II) + (III) → (IV) geeignete Basen sind insbesondere Alkalicarbonate wie Natrium-, Kalium- oder Cäsiumcarbonat, Alkali-Alkoholate wie Natrium- oder Kaliummethanolat, Natrium- oder Kaliumethanolat oder Natrium- oder Kalium-*tert*.-butylat, Alkalihydride wie Natrium- oder Kaliumhydrid, Amide wie Natriumamid, Lithium- oder Kalium-bis(trimethylsilyl)-amid oder Lithiumdiisopropylamid, oder metallorganische Verbindungen wie *n*-Butyllithium oder Phenyllithium. Bevorzugt wird Natrium-, Kalium- oder Cäsiumcarbonat als Base eingesetzt. Gegebenenfalls ist der Zusatz eines Alkylierungskatalysators, wie beispielsweise Lithiumbromid, Natrium- oder Kaliumiodid, Tetra-*n*-butylammoniumbromid oder Benzyltriethylammoniumchlorid, von Vorteil.

Die Umsetzung (II) + (III) → (IV) wird im Allgemeinen in einem Temperaturbereich von 0°C bis +150°C, bevorzugt bei +20°C bis +100°C durchgeführt.

Die Hydrolyse der Ester-Gruppen -C(O)OT¹ und -C(O)OT² im Verfahrensschritt (IV) → (I) wird nach üblichen Methoden durchgeführt, indem man die Ester in inerten Lösungsmitteln mit Säuren oder Basen behandelt, wobei bei letzterer Variante die zunächst entstehenden Salze durch Behandeln mit Säure in die freien Carbonsäuren überführt werden. Im Falle der *tert*.-Butylester erfolgt die Esterspaltung bevorzugt mit Säuren.

Bei unterschiedlichen Gruppen T¹ und T² kann die Hydrolyse gegebenenfalls simultan in einer Eintopf-Reaktion oder in zwei separaten Reaktionsschritten durchgeführt werden.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, *n*-Butanol oder *tert*.-Butanol, oder Ether wie Diethylether, Tetrahydrofuran, 1,4-Dioxan oder 1,2-Dimethoxyethan, oder andere Lösungsmittel wie Dichlormethan, Aceton, Methylethylketon, *N,N*-Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit Dioxan, Tetrahydrofuran, Methanol, Ethanol und/oder Dimethylformamid verwendet. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser eingesetzt.

Als Basen sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von -20°C bis +120°C, bevorzugt bei 0°C bis +80°C.

Die zuvor beschriebenen Verfahrensschritte können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Die Verbindungen der Formel (II) ihrerseits können beispielsweise dadurch hergestellt werden, dass man zunächst eine Keto-Verbindung der Formel (V) 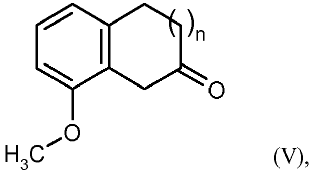 in welcher n die oben angegebene Bedeutung hat,
im Zuge einer reduktiven Aminierung mit einem 4-(Aminomethyl)benzoesäureester der Formel (VI) 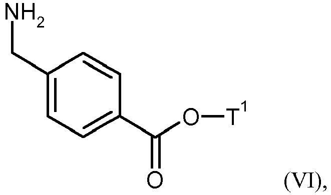 in welcher T¹ die oben angegebene Bedeutung hat,
zu einem sekundären Amin der Formel (VII) 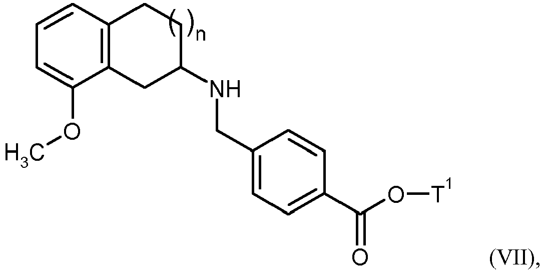 in welcher n und T¹ die oben angegebenen Bedeutungen haben,
umsetzt, nachfolgend in Gegenwart einer Base mit einem 5-Halovaleriansäureester der Formel (VIII) 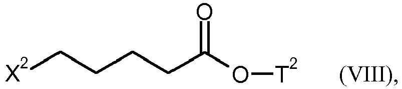 in welcher T² die oben angegebene Bedeutung hat
und
- X²: für Chlor, Brom oder Iod steht,
zu einem tertiären Amin der Formel (IX) 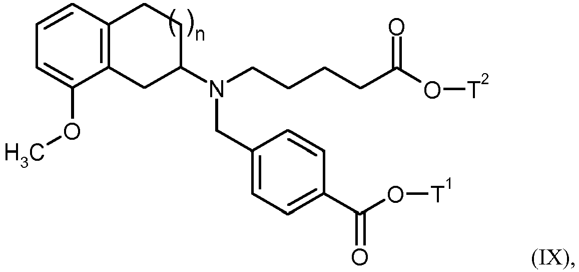 in welcher n, T¹ und T² jeweils die oben angegebenen Bedeutungen haben,
alkyliert und anschließend die phenolische Methylether-Gruppierung durch Behandlung mit Bortribromid oder Bromwasserstoff spaltet.

Die Umsetzung (V) + (VI) → (VII) erfolgt in den für eine reduktive Aminierung üblichen, unter den Reaktionsbedingungen inerten Lösungsmitteln, gegebenenfalls in Gegenwart einer Säure und/ oder eines wasserentziehenden Mittels als Katalysatoren. Zu diesen Lösungsmitteln gehören beispielsweise Wasser, Tetrahydrofuran, Dichlormethan, 1,2-Dichlorethan, *N,N*-Dimethylformamid und Alkohole wie Methanol, Ethanol, n-Propanol oder Isopropanol; auch ist es möglich, Gemische solcher Lösungsmittel einzusetzen. Bevorzugt werden Dichlormethan, Methanol oder Ethanol, jeweils unter Zusatz von Essigsäure, verwendet.

Als Reduktionsmittel für eine solche Aminierungsreaktion eignen sich insbesondere komplexe Borhydride, wie beispielsweise Natriumborhydrid, Natriumtriacetoxyborhydrid, Natriumcyanoborhydrid oder Tetra-*n*-butylammoniumborhydrid. Bevorzugt wird Natriumborhydrid oder Natriumtriacetoxyborhydrid eingesetzt.

Die Umsetzung (V) + (VI) → (VII) erfolgt im Allgemeinen in einem Temperaturbereich von -20°C bis +50°C, bevorzugt bei 0°C bis +30°C.

Die Alkylierung im Verfahrensschritt (VII) + (VIII) → (IX) wird unter analogen Reaktionsbedingungen bezüglich Lösungsmittel, Base und Temperatur durchgeführt, wie zuvor bei der Umsetzung (II) + (III) → (IV) beschrieben. Bevorzugt werden hier als Base Alkalicarbonate und als Lösungsmittel Acetonitril eingesetzt. Die Alkylierung erfolgt in der Regel in einem Temperaturbereich von +50°C bis +85°C.

Die Spaltung der phenolischen Methylether-Gruppe im Verfahrensschritt (IX) → (II) erfolgt nach üblichen Methoden durch Behandlung mit Bortribromid in Dichlormethan bei -20°C bis +10°C oder durch Erhitzen mit einer Lösung von Bromwasserstoff in Eisessig oder Wasser auf +100°C bis +120°C. Sollten unter den Reaktionsbedingungen gleichzeitig auch die Ester-Gruppierungen -C(O)OT¹ und -C(O)OT² ganz oder teilweise zu den entsprechenden freien Carbonsäuren der Formel (X) 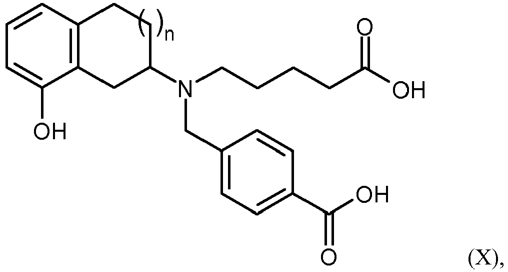 in welcher n die oben angegebene Bedeutung hat,
gespalten werden, so können diese beispielsweise durch nachfolgende Behandlung mit Thionylchlorid in Methanol oder Ethanol wieder rück-verestert werden.

Die zuvor beschriebenen Umsetzungen können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (II), (IV), (VII), (IX) oder (X) erfolgen, welche dann in separierter Form entsprechend den zuvor beschriebenen Verfahrenssequenzen weiter umgesetzt werden. Eine solche Auftrennung der Stereoisomeren läßt sich nach üblichen, dem Fachmann bekannten Methoden durchführen. Vorzugsweise werden chromatographische Verfahren an achiralen bzw. chiralen Trennphasen angewandt; im Falle von Carbonsäuren als Zwischen- oder Endprodukten kann alternativ auch eine Trennung über diastereomere Salze mit Hilfe chiraler Basen erfolgen.

Die Verbindungen der Formel (V) sind jeweils nach Literaturvorschriften zugänglich [siehe z.B. für 4-Methoxy-1,3-dihydro-2*H*-inden-2-on (*n* = *0*): S. Ghosh et al., Tetrahedron 1989, 45 (5), 1441-1446; für 8-Methoxy-3,4-dihydronaphthalin-2(1H)-on (*n* = *1*): N. T. Hatzenbuhler et al., WO 2005/012291-A1, Example 45; für 4-Methoxy-5,7,8,9-tetrahydro-6H-benzo[7]annulen-6-on (*n* = 2): U. Hacksell et al., J. Med. Chem. 1989, 32 (10), 2311-2318].

Die Verbindungen der Formeln (III), (VI) und (VIII) sind entweder kommerziell erhältlich oder als solche in der Literatur beschrieben, oder sie können auf für den Fachmann offenkundigem Wege in Analogie zu in der Literatur publizierten Methoden hergestellt werden. Zahlreiche detaillierte Vorschriften befinden sich auch im Experimentellen Teil im Abschnitt zur Herstellung der Ausgangsverbindungen und Intermediate.

Die Herstellung der erfindungsgemäßen Verbindungen kann durch das folgende Reaktionsschema beispielhaft veranschaulicht werden:

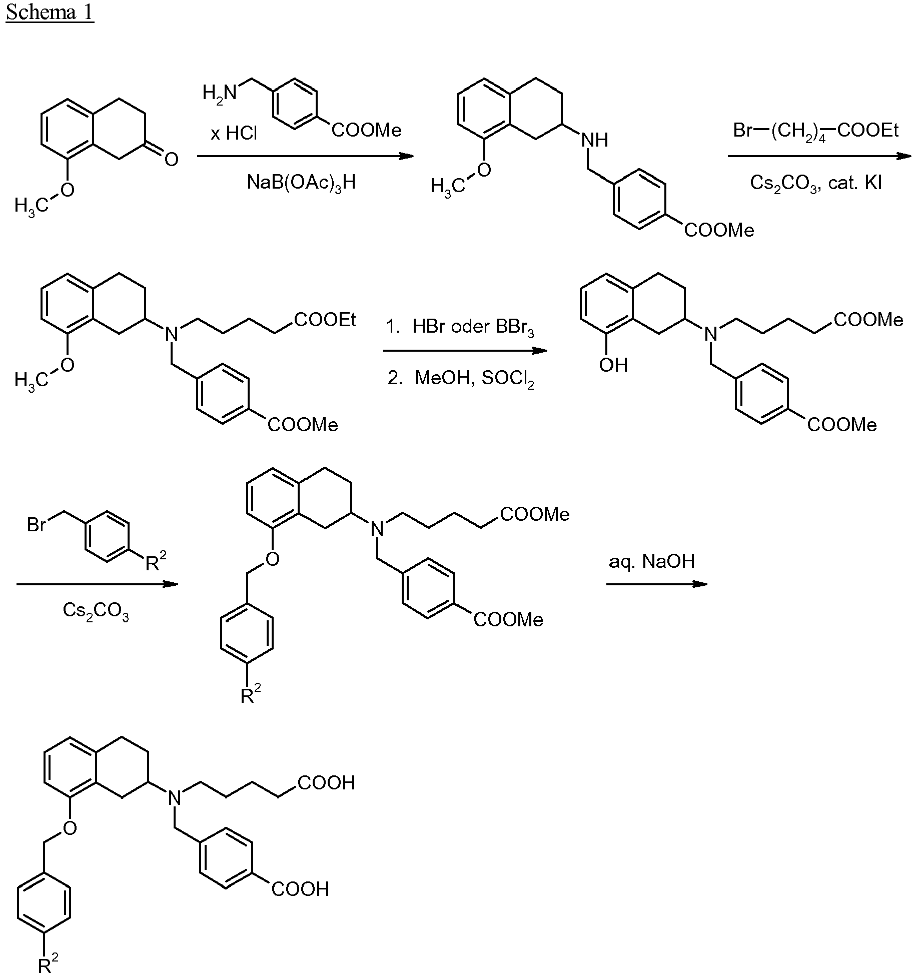

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente Aktivatoren der löslichen Guanylatcyclase dar. Sie führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte, Häm-unabhängige Aktivierung der löslichen Guanylatcyclase und einen intrazellulären cGMP-Anstieg vermittelt.

Die erfindungsgemäßen Verbindungen können daher in Arzneimitteln zur Behandlung von kardiovaskulären Erkrankungen wie beispielsweise des Bluthochdrucks und der Herzinsuffizienz, stabiler und instabiler Angina pectoris, pulmonaler Hypertonie, renaler Hypertonie, peripheren und kardialen Gefäßerkrankungen sowie Arrhythmien, zur Behandlung von thromboembolischen Erkrankungen und Ischämien wie Myokardinfarkt, Hirnschlag, transistorischen und ischämischen Attacken sowie peripheren Durchblutungsstörungen, zur Verhinderung von Restenosen wie nach Thrombolysetherapien, percutan-transluminalen Angioplastien (PTA), percutan-transluminalen Koronarangioplastien (PTCA) und Bypass, zur Behandlung von Arteriosklerose, asthmatischen Erkrankungen und Krankheiten des Urogenitalsystems wie beispielsweise überaktiver Blase, unterem Harntrakt-Syndrom (LUTS), Inkontinenz, Prostatahypertrophie, erektiler Dysfunktion und weiblicher sexueller Dysfunktion, sowie zur Behandlung von Osteoporose, Glaukom und Gastroparese eingesetzt werden.

Außerdem können die erfindungsgemäßen Verbindungen zur Behandlung von primärem und sekundärem Raynaud-Phänomen, von Mikrozirkulationsstörungen, Claudicatio, Tinnitus, peripheren und autonomen Neuropathien, diabetischen Mikroangiopathien, diabetischer Retinopathie, diabetischen Geschwüren an den Extremitäten, CREST-Syndrom, Erythematose, Onychomykose sowie von rheumatischen Erkrankungen verwendet werden.

Die erfindungsgemäßen Verbindungen können darüber hinaus zur Verhinderung von ischämie-und/oder reperfusionsbedingten Schädigungen von Organen oder Geweben sowie als Zusatzstoffe für Perfusions- und Konservierungslösungen von Organen, Organteilen, Geweben oder Gewebeteilen menschlichen oder tierischen Ursprungs insbesondere bei chirurgischen Eingriffen oder im Bereich der Transplantationsmedizin Verwendung finden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen anti-inflammatorische Wirkung und können daher als entzündungshemmende Mittel eingesetzt werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin All-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin All-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin All-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht. Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- aq.: wässrig, wässrige Lösung
- ATP: Adenosin-5'-triphosphat
- Brij^{®}: Polyethylenglycoldodecylether
- BSA: bovines Serumalbumin
- Bsp.: Beispiel
- c: Konzentration
- cat.: katalytisch
- DMF: Dimethylformamid
- DMSO: Dimethylsulfoxid
- d. Th.: der Theorie (bei Ausbeute)
- DTT: Dithiothreitol
- ee: Enantiomerenüberschuss
- *ent*: enantiomerenrein, Enantiomer
- eq.: Äquivalent(e)
- ESI: Elektrospray-Ionisation (bei MS)
- Et: Ethyl
- GTP: Guanosin-5'-triphosphat
- h: Stunde(n)
- HPLC: Hochdruck-, Hochleistungsflüssigchromatographie
- LC-MS: Flüssigchromatographie-gekoppelte Massenspektroskopie
- Me: Methyl
- min: Minute(n)
- MS: Massenspektroskopie
- NMR: Kernresonanzspektroskopie
- *rac*: racemisch, Racemat
- RP: reverse phase (Umkehrphase, bei HPLC)
- RT: Raumtemperatur
- Rₜ: Retentionszeit (bei HPLC)
- s.o.: siehe oben
- TEA: Triethanolamin
- TFA: Trifluoressigsäure
- THF: Tetrahydrofuran
- UV: Ultraviolett-Spektroskopie
- v/v: Volumen zu Volumen-Verhältnis (einer Lösung)
- zus.: zusammen

### HPLC- und LC-MS-Methoden:

### Methode 1 (präparative HPLC):

Säule: Grom-Sil C18 10 µm, 250 mm x 30 mm; Eluent A: Wasser + 0.1% Ameisensäure, Eluent B: Acetonitril; Programm: 0-5 min 10% B, 5-38 min Gradient bis 95% B; Fluss: 50 ml/min; UV-Detektion: 210 nm.

### Methode 2 (LC-MS):

Instrument: Waters Acquity SQD UPLC System; Säule: Waters Acquity UPLC HSS T3 1.8 µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 3 (LC-MS):

Gerätetyp MS: Waters Micromass Quattro Micro; Gerätetyp HPLC: Agilent 1100 Serie; Säule: Thermo Hypersil GOLD 3 µ, 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Instrument: Micromass Quattro Premier mit Waters UPLC Acquity; Säule: Thermo Hypersil GOLD 1.9 µ, 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 5 (chirale analytische HPLC):

Stationäre Phase: Daicel OD-H; Säule: 250 mm x 4 mm; UV-Detektion: 230 nm; Elutionsmittel: Isopropanol/Isohexan 30:70 (v/v); Fluss: 1.0 ml/min.

### Methode 6 (chirale analytische HPLC):

Stationäre Phase: Daicel Chiralpak IA; Säule: 250 mm x 4 mm; UV-Detektion: 230 nm; Elutionsmittel: Ethanol/Methyl-*tert*.-butylether 75:25 (v/v); Fluss: 1.0 ml/min.

### Methode 7 (präparative LC-MS):

Instrument MS: Waters, Instrument HPLC: Waters; Säule: Waters X-Bridge C18 5 µm, 18 mm x 50 mm; Eluent A: Wasser + 0.05% Triethylamin, Eluent B: Acetonitril + 0.05% Triethylamin; Gradient: 0.0 min 95% A → 0.15 min 95% A → 8.0 min 5% A → 9.0 min 5% A; Fluss: 40 ml/ min; UV-Detektion: DAD, 210-400 nm.

### Methode 8 (präparative LC-MS):

Instrument MS: Waters, Instrument HPLC: Waters; Säule: Phenomenex Luna 5 µ C18(2) 100A, 50 mm x 21.2 mm; Eluent A: Wasser + 0.05% Ameisensäure, Eluent B: Acetonitril + 0.05% Ameisensäure; Gradient: 0.0 min 95% A → 0.15 min 95% A → 8.0 min 5% A → 9.0 min 5% A; Fluss: 40 ml/min; UV-Detektion: DAD, 210-400 nm.

### Methode 9 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril + 0.025% Ameisensäure; Gradient: 0.0 min 98% A → 0.9 min 25% A → 1.0 min 5% A → 1.4 min 5% A → 1.41 min 98% A → 1.5 min 98% A; Ofen: 40°C; Fluss: 0.60 ml/min; UV-Detektion: DAD, 210 nm.

### Ausgangsverbindungen und Intermediate:

### Beispiel 1A

### rac-Methyl-4-{[(8-methoxy-1,2,3,4-tetrahydronaphthalin-2-yl)amino]methyl}benzoat

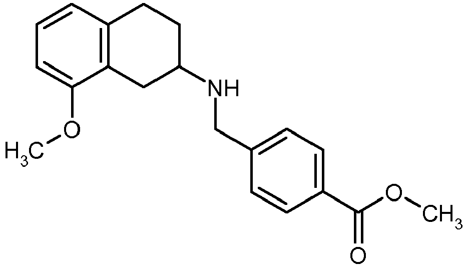

15 g (74.4 mmol) 4-(Aminomethyl)benzoesäuremethylester-Hydrochlorid, 13.8 g (78.1 mmol) 8-Methoxy-3,4-dihydronaphthalin-2(1*H*)-on [zur Herstellung siehe WO 2005/012291-A1, Example 45], 14.3 ml (81.8 mmol) *N,N*-Diisopropylethylamin, 4.7 ml Essigsäure und 20.5 g (96.7 mmol) Natriumtriacetoxyborhydrid wurden in 600 ml Dichlormethan suspendiert und über Nacht bei RT gerührt. Die Reaktionsmischung wurde danach eingeengt und der Rückstand mit Ethylacetat und Wasser 1 h bei RT gerührt. Der hierbei ausgefallene Feststoff wurde abgesaugt. Die Filtrat-Phasen wurden getrennt und die wässrige Phase zweimal mit Ethylacetat extrahiert. Die vereinigten organischen Phasen wurden getrocknet und eingeengt. Der oben erhaltene Feststoff (18.2 g) und der Rückstand der organischen Phase (12.9 g) wurden vereinigt und erneut in einem Gemisch aus Dichlormethan, Wasser und gesättigter Kaliumcarbonat-Lösung (pH 12) bis zur kompletten Auflösung gerührt. Die organische Phase wurde anschließend abgetrennt, getrocknet und eingeengt. Die Titelverbindung wurde in Form des Rückstands erhalten.

Ausbeute: 20.4 g (83% d. Th.)

LC-MS (Methode 2): Rₜ = 0.71 min; MS (ESIpos): m/z = 326 [M+H]⁺

¹H-NMR (400 MHz, DMSO-*d*₆): δ [ppm] = 7.90 (d, 2H), 7.52 (d, 2H), 7.04 (t, 1H), 6.71 (d, 1H), 6.65 (d, 1H), 3.79-3.96 (m, 5H), 3.74 (s, 3H), 2.86-3.01 (m, 1H), 2.71-2.86 (m, 2H), 2.57-2.71 (m, 1H), 2.10-2.41 (m, 2H), 1.85-2.05 (m, 1H), 1.34-1.58 (m, 1H).

### Beispiel 2A

### rac-Methyl-4-{[(5-ethoxy-5-oxopentyl)(8-methoxy-1,2,3,4-tetrahydronaphthalin-2-yl)amino]-methyl}benzoat

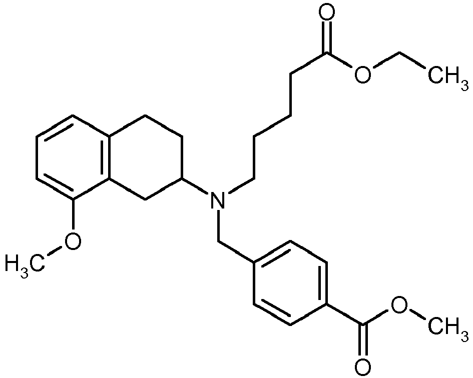

Unter Argon wurden 13.4 g (41.2 mmol) der Verbindung aus Beispiel 1A in 160 ml Acetonitril gelöst, mit 11.8 ml (15.5 g, 74.1 mmol) 5-Bromvaleriansäureethylester, 27 g (82.4 mmol) Cäsiumcarbonat sowie 685 mg (4.1 mmol) Kaliumiodid versetzt und über Nacht unter Rückfluss gerührt. Nach Zugabe von weiteren 550 mg Kaliumiodid wurde erneut über Nacht unter Rückfluss gerührt. Anschließend wurden nochmals 2 g Kaliumiodid zugesetzt und wiederum über Nacht unter Rückfluss gerührt. Nach dem Abkühlen wurde vom Niederschlag abfiltriert und das Filtrat eingeengt. Dieser Rückstand wurde dann an 1.2 kg Kieselgel mit Isohexan/Ethylacetat als Laufmittel (Gradient 10:1 1 → 5:1) chromatographisch gereinigt. Es wurden 10.95 g (59% d. Th.) der Titelverbindung als farbloser Feststoff erhalten.

LC-MS (Methode 2): Rₜ = 0.86 min; MS (ESIpos): m/z = 454 [M+H]⁺

¹H-NMR (400 MHz, DMSO-*d*₆): δ [ppm] = 7.90 (d, *J* = 8.07 Hz, 2H), 7.50 (d, *J* = 8.07 Hz, 2H), 7.04 (t, *J* = 7.83 Hz, 1H), 6.52-6.81 (m, 2H), 4.00 (q, *J* = 7.09 Hz, 2H), 3.83 (s, 3H), 3.61-3.80 (m, 5H), 2.82 (d, *J* = 15.41 Hz, 3H), 2.70 (br. s, 1H), 2.18 (t, *J* = 7.21 Hz, 2H), 1.87-2.04 (m, 1H), 1.28-1.65 (m, 4H), 1.13 (t, *J* = 7.09 Hz, 3H).

### Beispiel 3A

### rac-Methyl-4-{[(8-hydroxy-1,2,3,4-tetrahydronaphthalin-2-yl)(5-methoxy-5-oxopentyl)amino]-methyl}benzoat

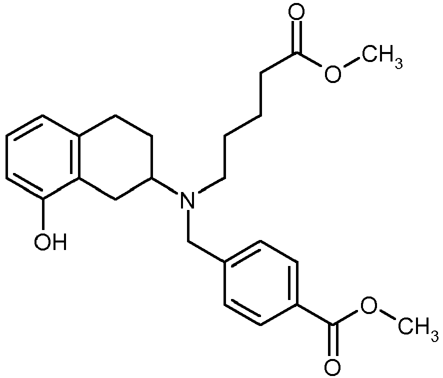

Unter Argon wurden zu einer Lösung von 1.2 g (2.65 mmol) der Verbindung aus Beispiel 2A in 35 ml Dichlormethan bei 0°C 3 ml (3 mmol) einer 1 N Lösung von Bortribromid in Dichlormethan getropft und die Mischung 1 h bei 0°C gerührt. Danach wurden weitere 6 ml (6 mmol) der Bortribromid-Lösung zugetropft und nochmals 45 min nachgerührt (zum Ende der Zugabe bildete sich ein heller Niederschlag). Dann wurden 35 ml Methanol zugetropft, die entstandene Lösung 3 h lang unter Rückfluss erhitzt und schließlich eingeengt. Der Rückstand wurde in 80 ml Methanol gelöst, mit 0.2 ml Thionylchlorid versetzt und 4 h unter Rückfluss erhitzt. Anschließend wurde erneut eingeengt. Es verblieben 1.15 g der Titelverbindung als Rohprodukt, das in dieser Form weiter umgesetzt wurde.

LC-MS (Methode 4): Rₜ = 0.86 min; MS (ESIpos): m/z = 426 [M+H]⁺.

Sauberes Material für die NMR-Spektroskopie wurde durch Kieselgel-Chromatographie einer Probe mit einem Eluent-Gradienten aus Dichlormethan und 0-12% Methanol erhalten.

¹H-NMR (400 MHz, DMSO-*d*₆): δ [ppm] = 9.18 (s, 1H), 7.85-7.96 (m, 2H), 7.51 (m, 2H), 6.85 (t, 1H), 6.56 (d, 1H), 6.48 (d, 1H), 3.83 (s, 3H), 3.73 (q, 2H), 3.54 (s, 3H), 2.60-2.93 (m, 4H), 2.30-2.45 (m, 2H), 2.20 (t, 2H), 1.88-1.97 (m, 1H), 1.28-1.75 (m, 6H).

### Beispiel 4A

### rac-4-{[(5-Ethoxy-5-oxopentyl)(8-hydroxy-1,2,3,4-tetrahydronaphthalin-2-yl)amino]methyl}benzoesäureethylester-Hydrochlorid

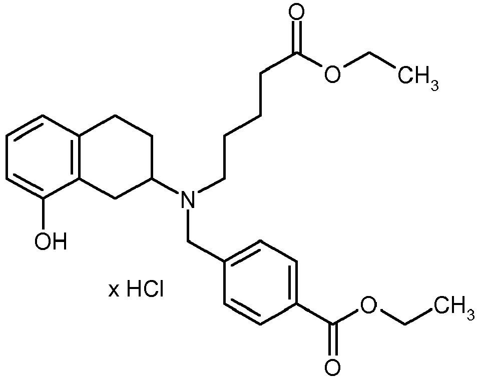

8.8 g (20.7 mmol) der Verbindung aus Beispiel 3A wurden in 270 ml THF und 130 ml Methanol gelöst und nach Zugabe von 25.5 ml 5 N Natronlauge über Nacht bei RT gerührt. Danach wurde mit 27 ml 5 N Salzsäure angesäuert, das Gemisch im Vakuum eingeengt und der Rückstand im Hochvakuum weiter getrocknet. Der Rückstand (15.7 g, nach LC-MS noch Edukt-Material enthaltend) wurde in Ethanol gelöst, erneut mit 25.5 ml 5 N Natronlauge versetzt und 1 h unter Rückfluss gerührt. Dann wurde wieder eingeengt und der Rückstand zweimal mit Ethanol co-destilliert. Der verbliebene Rückstand enthielt nach LC-MS (Methode 2) zu 79% die Dicarbonsäure 4-{[(4-Carboxybutyl)(8-hydroxy-1,2,3,4-tetrahydronaphthalin-2-yl)amino]methyl}benzoesäure (Rₜ = 0.61 min; MS (ESIpos): m/z = 398 [M+H]⁺).

Dieser Rückstand wurde in 210 ml Ethanol teilweise gelöst, tropfenweise mit 1.9 ml Thionylchlorid versetzt und 6 h bei 65°C gerührt. Es wurde mit weiterem Ethanol verdünnt, bis der Ansatz wieder rührfähig wurde, und erneut 6 h bei 65°C gerührt. Nach Zugabe von weiteren 10 ml Thionylchlorid wurde nochmals über Nacht bei 65°C nachgerührt. Nach dem Abkühlen wurde von anorganischem Material abgesaugt und der Rückstand mit Ethanol nachgewaschen. Das eingeengte Filtrat (ca. 17 g) wurde mit ca. 100 ml Ethanol versetzt, gut durchgeschüttelt und dann erneut abgesaugt. Der Feststoff wurde mit ca. 50 ml Ethanol nachgewaschen und im Vakuum bei 40°C getrocknet.

Ausbeute: 4.3 g eines bräunlichen Feststoffs

LC-MS (Methode 2): Rₜ = 0.86 min; MS (ESIpos): m/z = 454 [M+H]⁺

¹H-NMR (400 MHz, DMSO-*d*₆): δ [ppm] = 10.32-10.57 (br, 1H), 9.52-9.65 (m, 1H), 7.98-8.09 (m, 2H), 7.76-7.93 (m, 2H), 6.88-7.01 (m, 1H), 6.60-6.71 (m, 1H), 6.48-6.61 (m, 1H), 4.22-4.77 (m, 4H), 3.94-4.09 (m, 2H), 3.49-3.73 (m, 1H), 2.95-3.28 (m, 3H), 2.60-2.93 (m, 3H), 2.14-2.45 (m, 3H), 1.38-2.00 (m, 6H), 1.25-1.38 (m, 3H), 1.08-1.20 (m, 3H).

### Beispiel 5A

### rac-Methyl-4-{[{8-[(4-tert.-butylbenzyl)oxy]-1,2,3,4-tetrahydronaphthalin-2-yl}(5-methoxy-5-oxo-pentyl)amino]methyl}benzoat

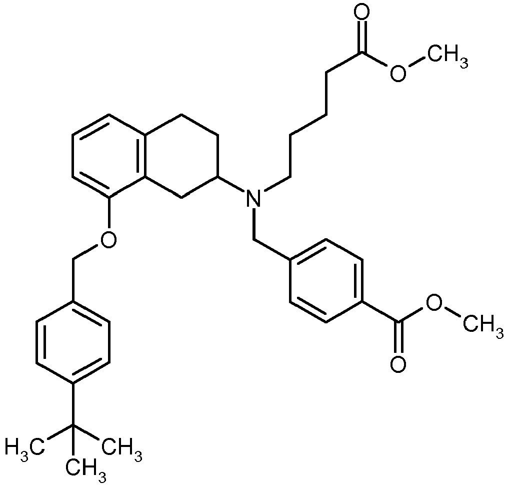

550 mg (1.29 mmol) der Verbindung aus Beispiel 3A wurden in 35 ml DMF gelöst, mit 320 µl (1.6 mmol) 4-*tert*.-Butylbenzylbromid sowie 1.4 g (4.14 mmol) Cäsiumcarbonat versetzt und 18 h bei RT gerührt. Das Gemisch wurde danach mit Wasser versetzt und mehrfach mit Ethylacetat extrahiert. Die vereinigten organischen Phasen wurden über Natriumsulfat getrocknet und eingeengt. Das erhaltene Rohprodukt wurde durch präparative HPLC (Methode 1) gereinigt.

Ausbeute: 230 mg (31% d. Th.)

LC-MS (Methode 3): Rₜ = 2.32 min; MS (ESIpos): m/z = 572 [M+H]⁺.

### Beispiel 6A und Beispiel 7A

### ent-Methyl-4-{[{8-[(4-tert.-butylbenzyl)oxy]-1,2,3,4-tetrahydronaphthalin-2-yl}(5-methoxy-5-oxo-pentyl)amino]methyl}benzoat (Enantiomer 1 und 2)

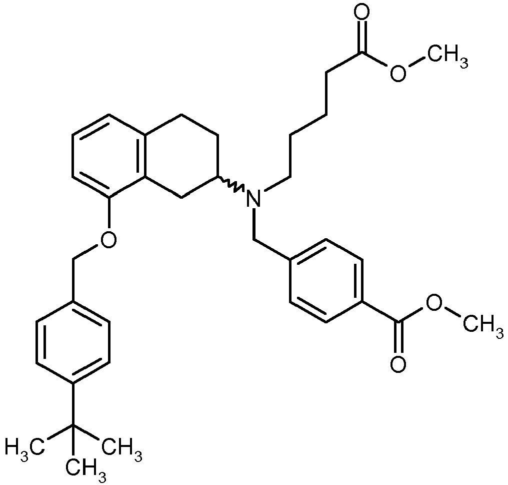

160 mg des racemischen Methyl-4-{[{8-[(4-*tert*.-butylbenzyl)oxy]-1,2,3,4-tetrahydronaphthalin-2-yl}(5-methoxy-5-oxopentyl)amino]methyl}benzoats aus Beispiel 5A wurden mittels präparativer HPLC an chiraler Phase in die Enantiomere aufgetrennt [Probenvorbereitung: die Substanz wurde in 10 ml Isopropanol gelöst und die Lösung mit 10 ml Hexan versetzt; Injektionsvolumen: je 1 ml; Säule: Daicel Chiralpak OD-H, 250 mm x 20 mm; Elutionsmittel: Isohexan/Isopropanol 80:20 (v/v); Fluss: 18 ml/min; UV-Detektion: 230 nm; Temperatur: RT]:

### Beispiel 6A (Enantiomere 1):

Ausbeute: 34 mg

LC-MS (Methode 4): Rₜ = 1.43 min; MS (ESIpos): m/z = 572 [M+H]⁺

HPLC (Methode 5): Rₜ = 5.28 min, 99.5% ee

¹H-NMR (400 MHz, DMSO-*d*₆): δ [ppm] = 7.83-7.98 (m, 2H), 7.27-7.57 (m, 6H), 6.95-7.07 (m, 1H), 6.74-6.84 (m, 1H), 6.58-6.70 (m, 1H), 5.05 (s, 2H), 3.65-3.83 (m, 5H), 3.53 (s, 3H), 2.61-2.99 (m, 4H), 2.14-2.26 (m, 2H), 1.87-2.05 (m, 1H), 1.45-1.65 (m, 3H), 1.34-1.44 (m, 2H), 1.29 (s, 9H).

### Beispiel 7A (Enantiomer 2):

Ausbeute: 31 mg

LC-MS (Methode 4): Rₜ = 1.43 min; MS (ESIpos): m/z = 572 [M+H]⁺

HPLC (Methode 5): Rₜ = 6.02 min, 96.7% ee.

Analog zur Vorschrift für Beispiel 5A wurden die folgenden Verbindungen ausgehend von *rac-*Methyl-4-{[(8-hydroxy-1,2,3,4-tetrahydronaphthalin-2-yl)(5-methoxy-5-oxopentyl)amino]methyl}-benzoat und dem jeweils aufgeführten Alkylhalogenid hergestellt:

| **Beispiel** | **Struktur** | **Edukt** | **Ausbeute; analytische Daten** |
|---|---|---|---|
| **8A** | 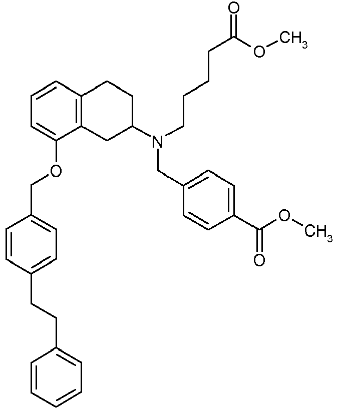 | 1-(Chlormethyl)-4-(2-phenylethyl)-benzol | 14% d. Th.; |
| | | | LC-MS (Methode 2): Rₜ = 1.22 min, |
| | | | m/z = 620 [M+H]⁺ |
| | (*Racemat*) | | |
| **9A** | 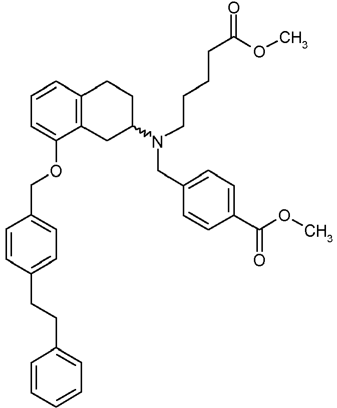 | Beispiel 8A¹⁾ | 34% d. Th.; LC-MS (Methode 2): Rₜ = 1.19 min, |
| | | | m/z = 620 [M+H]⁺; |
| | | | HPLC (Methode 6): Rₜ = 4.56 min, >99.5% ee |
| | (*Enantiomer 1*) | | |
| **10A** | 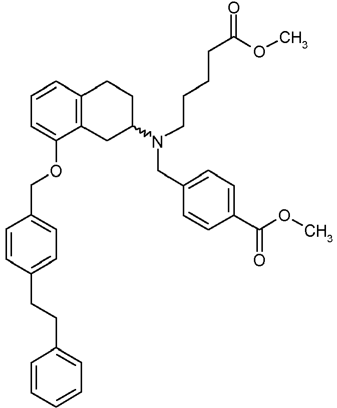 | Beispiel 8A¹⁾ | 35% d. Th.; |
| | | | LC-MS (Methode 2): Rₜ = 1.18 min, |
| | | | m/z = 620 [M+H]⁺; |
| | | | HPLC (Methode 6): Rₜ = 5.26 min, 96.8% ee |
| | (*Enantiomer 2*) | | |
| **11A** | 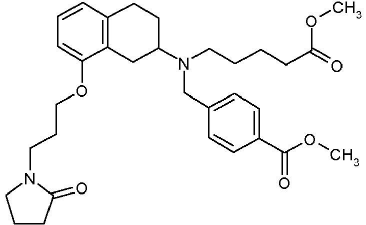 | 1-(3-Chlor-propyl)-pyrrolidin-2-on | 10% d. Th.; |
| | | | LC-MS (Methode 2): Rₜ = 0.79 min, |
| | | | m/z = 551 [M+H]⁺ |
| | (*Racemat*) | | |
| **12A** | 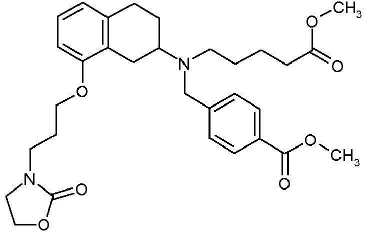 | 3-(3-Chlor-propyl)-1,3-oxazolidin-2-on | 55% d. Th.; |
| | | | LC-MS (Methode 4): Rₜ = 0.93 min, |
| | | | m/z = 553 [M+H]⁺ |
| | (*Racemat*) | | |
| **13A** | 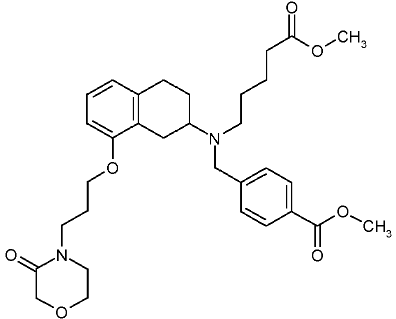 | 4-(3-Brom-propyl)-morpholin-3-on | 14% d. Th.; |
| | | | LC-MS (Methode 2): Rₜ = 0.84 min, |
| | | | m/z = 567 [M+H]⁺ |
| | (*Racemat*) | | |

| | | | |
|---|---|---|---|
| ¹⁾ Methode Enantiomerentrennung: | | | |

Probenvorbereitung: 100 mg des Racemats wurden in 10 ml Isopropanol gelöst und die Lösung mit 10 ml Hexan versetzt; Injektionsvolumen: je 0.1 ml; Säule: Daicel Chiralpak IA, 250 mm x 20 mm; Elutionsmittel: Ethanol/Methyl-*tert*.-butylether 75:25 (v/v); Fluss: 18 ml/min; UV-Detektion: 230 nm; Temperatur: RT.

### Ausführungsbeispiele:

### Beispiel 1

### rac-4-{[{8-[(4-tert.-Butylbenzyl)oxy]-1,2,3,4-tetrahydronaphthalin-2-yl}(4-carboxybutyl)amino]-methyl}benzoesäure

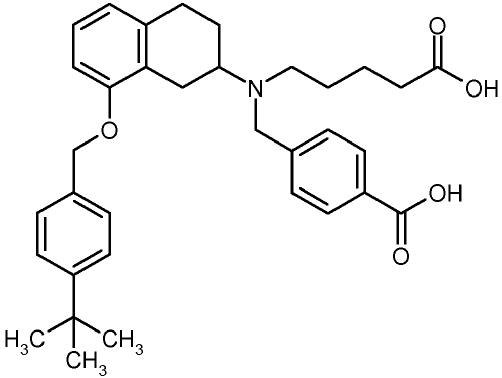

68.5 mg (0.12 mmol) der Verbindung aus Beispiel 5A wurden in 0.5 ml Methanol und 1 ml Dioxan gelöst, mit 0.15 ml 45%-iger Natronlauge und 0.2 ml Wasser versetzt und dann 45 min bei 100°C Badtemperatur gerührt. Die milchige Suspension wurde danach mit Wasser verdünnt, mit 2 N Salzsäure angesäuert und mehrfach mit Dichlormethan extrahiert. Die vereinigten organischen Phasen wurden über Natriumsulfat getrocknet und eingeengt. Der Rückstand wurde durch präparative HPLC (Methode 1) gereinigt.

Ausbeute: 50.5 mg (76% d. Th.)

LC-MS (Methode 2): Rₜ = 1.01 min; MS (ESIpos): m/z = 544 [M+H]⁺

¹H-NMR (500 MHz, DMSO-*d*₆): δ [ppm] = 7.92 (d, 2H), 7.28-7.63 (m, 6H), 7.04 (t, 1H), 6.82 (d, 1H), 6.68 (d, 1H), 4.95-5.20 (m, 2H), 5.08 (s, 2H), 3.69-3.92 (m, 2H), 2.65-3.01 (m, 4H), 2.18 (br. s, 2H), 1.87-2.08 (m, 1H), 1.39-1.70 (m, 5H), 1.32 (s, 9H).

Analog zur Vorschrift für Beispiel 1 wurden die folgenden Verbindungen hergestellt:

| **Beispiel** | **Struktur** | **Edukt** | **Ausbeute; analytische Daten** |
|---|---|---|---|
| **2** | 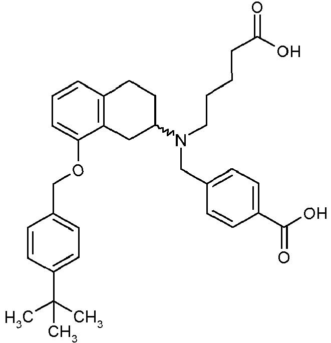 | 6A | 73% d. Th.; |
| | | | LC-MS (Methode 2): Rₜ = 1.01 min, |
| | | | m/z = 544 [M+H]⁺ |
| | (*Enantiomer 1*) | | |
| **3** | 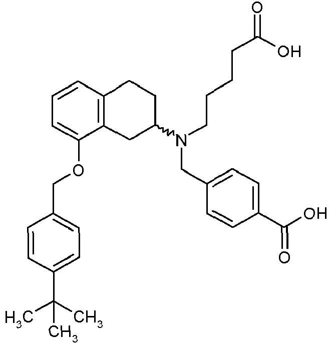 | 7A | 28% d. Th.; |
| | | | LC-MS (Methode 2): Rₜ = 1.02 min, |
| | | | m/z = 544 [M+H]⁺ |
| | (*Enantiomer 2)* | | |
| **4** | 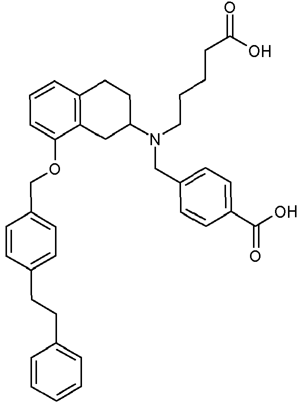 | 8A¹⁾ | 87% d. Th.; |
| | | | LC-MS (Methode 4): Rₜ = 1.27 min, |
| | | | m/z = 592 [M+H]⁺ |
| | (*Racemat*) | | |
| **5** | 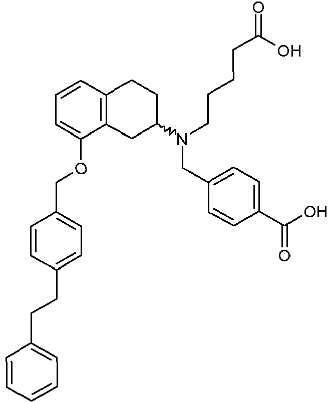 | 9A | 72% d. Th.; |
| | | | LC-MS (Methode 2): Rₜ = 1.05 min, |
| | | | m/z = 592 [M+H]⁺; |
| | | | HPLC (Methode 6): Rₜ = 4.56 min, >99.5% ee |
| | (*Enantiomer 1*) | | |
| **6** | 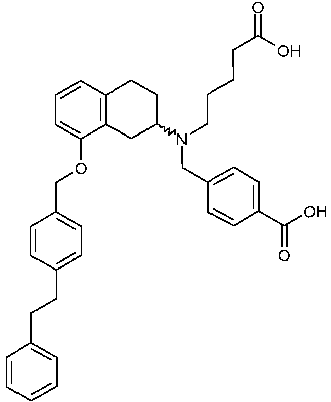 | 10A | 68% d. Th.; |
| | | | LC-MS (Methode 2): Rₜ = 1.05 min, |
| | | | m/z = 592 [M+H]⁺; HPLC (Methode 6): Rₜ = 5.26 min, 96.8% ee |
| | | | ¹H-NMR (400 MHz, DMSO-*d*₆): δ [ppm] = 7.88 (d, 2H), 7.48 (d, 2H), 7.12-7.37 (m, 9H), 6.93-7.06 (m, 1H), 6.78 (d, 1H), 6.65 (d, 1H), 5.05 (s, 2H), 3.74 (dd, 2H), 2.62-2.95 (m, 8H), 2.15 (t, 2H), 1.99 (m, 1H), 1.31-1.68 (m, 5H). |
| | (*Enantiomer 2*) | | |
| **7** | 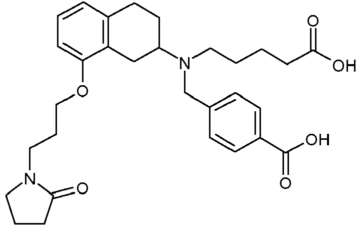 O OH N O OH O N O | 11A¹⁾³⁾ | 84% d. Th.; |
| | | | LC-MS (Methode 2): Rₜ = 0.75 min, |
| | | | m/z = 523 [M+H]⁺ |
| | (*Racemat*) | | |
| **8** | 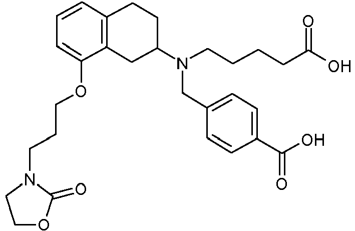 | 12A²⁾ | 21% d. Th.; |
| | | | LC-MS (Methode 2): Rₜ = 0.72 min, |
| | | | m/z = 525 [M+H]⁺ |
| | (*Racemat*) | | |
| **9** | 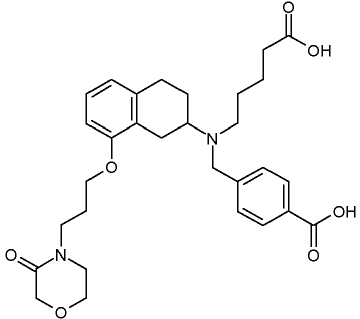 | 13A¹⁾⁴⁾ | 79% d. Th.; |
| | | | LC-MS (Methode 2): Rₜ = 0.72 min, |
| | | | m/z = 539 [M+H]⁺ |
| | (*Racemat*) | | |

| | | | |
|---|---|---|---|
| ¹⁾ Zur Aufarbeitung wurde hier das Reaktionsgemisch mit verdünnter Ameisensäure versetzt und direkt über präparative HPLC gereinigt. ²⁾ Die Ester-Hydrolyse wurde bei einer Badtemperatur von 50°C durchgeführt. ³⁾ Die Ester-Hydrolyse wurde bei einer Badtemperatur von 70°C durchgeführt. ⁴⁾ Die Ester-Hydrolyse wurde bei Raumtemperatur durchgeführt. | | | |

### Allgemeine Vorschrift für die Herstellung weiterer Ausführungsbeispiele mittels Parallelsynthese:

Jeweils 1.2 Äquivalente (0.12 mmol) des betreffenden Alkylhalogenids wurden in einer Vertiefung einer 96er "deep well"-Mikrotiterplatte vorgelegt und mit einer Lösung von 47 mg (0.1 mmol) der Verbindung aus Beispiel 4A in 0.6 ml DMF versetzt. Zu diesem Gemisch wurden 44 mg (0.32 mmol) Kaliumcarbonat gegeben. Die Mikrotiterplatte wurde abgedeckt und über Nacht bei 80°C geschüttelt. Dann wurde abfiltriert, das Filtrat mit 0.6 ml 4 N Natronlauge versetzt, erneut abgedeckt und bei 60°C über Nacht geschüttelt. Danach wurde das Lösungsmittel abgedampft. Der Rückstand wurde in 0.6 ml DMSO aufgenommen und direkt über präparative LC-MS gereinigt (Methode 7 oder 8). Die produkthaltigen Fraktionen wurden mittels Zentrifugaltrockner im Vakuum eingeengt. Der Rückstand der einzelnen Fraktionen wurde in je 0.6 ml DMSO gelöst und vereinigt. Abschließend wurde im Zentrifugaltrockner das Lösungsmittel vollständig abgedampft.

Nach dieser Vorschrift wurden die folgenden Verbindungen erhalten:

| **Beispiel** | **Struktur** | **LC-MS (Methode 9)** |
|---|---|---|
| **10** | 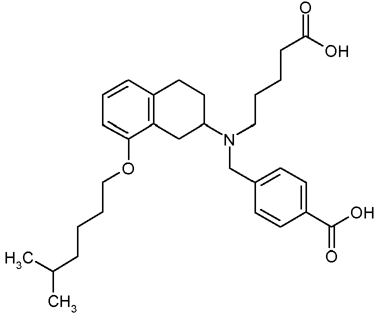 | Rₜ = 1.01 min, m/z = 496 [M+H]⁺ |
| | (*Racemat*) | |
| **11** | 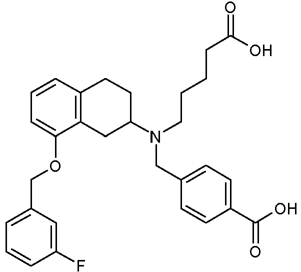 | Rₜ = 0.93 min, m/z = 506 [M+H]⁺ |
| | (*Racemat*) | |
| **12** | 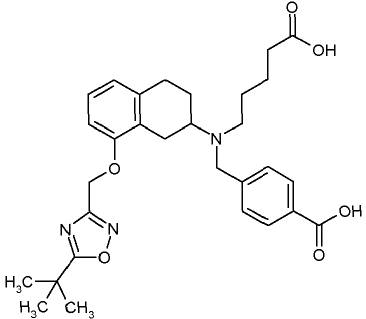 | Rₜ = 0.90 min, m/z = 536 [M+H]⁺ |
| | (*Racemat*) | |
| **13** | 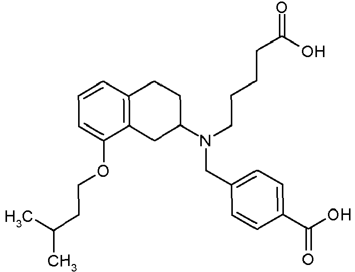 | Rₜ = 0.95 min, m/z = 468 [M+H]⁺ |
| | (*Racemat*) | |
| **14** | 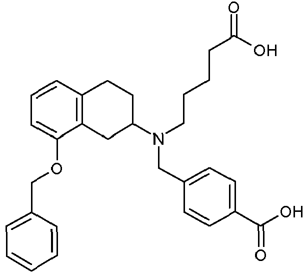 | Rₜ = 0.92 min, m/z = 486 [M+H]⁺ |
| | (*Racemat*) | |
| **15** | 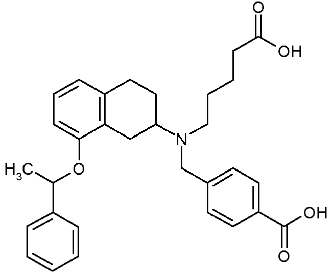 | Rₜ = 0.93 min, m/z = 502 [M+H]⁺ |
| | (*Racemat*) | |
| **16** | 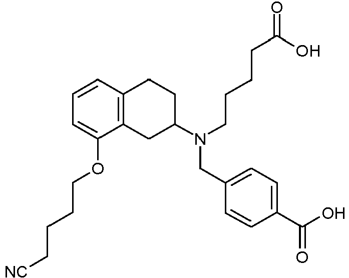 | Rₜ = 0.85 min, m/z = 479 [M+H]⁺ |
| | (*Racemat*) | |
| **17** | 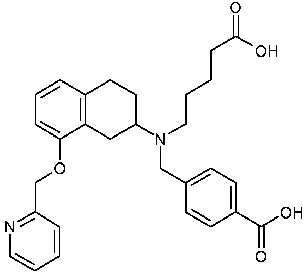 | Rₜ = 0.80 min, m/z = 489 [M+H]⁺ |
| | (*Racemat*) | |

### B. Bewerteng der pharmakologischen Wirksamkeit

Die pharmakologische Wirkung der erfindungsgemäßen Verbindungen kann in folgenden Assays gezeigt werden:

### B-1. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Ergebnisse zu den erfindungsgemäßen Verbindungen sind in Tabelle 1 aufgeführt:

**Tabelle 1: sGC-aktivierende Wirkung in der CHO-Reporterzelle in vitro**

| **Beispiel Nr.** | **MEC [nM]** |
|---|---|
| 1 | 0.3 |
| 4 | 3.0 |
| 5 | 0.3 |
| 10 | 30 |
| 11 | 300 |

| | |
|---|---|
| (MEC = minimale effektive Konzentration). | |

### B-2. Stimulation der sGC-Enzymaktivität

Lösliche Guanylatcyclase (sGC) setzt unter Stimulation GTP zu cGMP und Pyrophosphat (PPi) um. PPi wird mit Hilfe des nachfolgend beschriebenen Tests nachgewiesen. Das im Test entstehende Signal nimmt mit fortschreitender Umsetzung zu und dient als Maß für die sGC-Enzymaktivität unter der gegebenen Stimulation.

Zur Durchführung des Tests werden 29 µl Enzymlösung [0-10 nM lösliche Guanylatcyclase (hergestellt nach Hönicka et al., J. Mol. Med. 77, 14-23 (1999)) in 50 mM TEA, 2 mM MgCl₂, 0.1% BSA (Fraktion V), 0.005% Brij^{®}, pH 7.5] in eine Mikroplatte vorgelegt und 1 µl der zu testenden Substanz (als seriell verdünnte Lösung in DMSO) hinzugegeben. Der Ansatz wird 10 min bei Raumtemperatur inkubiert. Anschließend werden 20 µl Detektionsmix [1.2 nM Firefly-Luciferase (*Photinus pyralis*-Luciferase, Fa. Promega), 29 µM Dehydro-Luziferin (hergestellt nach Bitler & McElroy, Arch. Biochem. Biophys. 72, 358 (1957)), 122 µM Luziferin (Fa. Promega), 153 µM ATP (Fa. Sigma) und 0.4 mM DTT (Fa. Sigma) in 50 mM TEA, 2 mM MgCl₂, 0.1% BSA (Fraktion V), 0.005% Brij^{®}, pH 7.5] zugegeben. Die Enzymreaktion wird durch Zugabe von 20 µl Substratlösung [1.25 mM Guanosin-5'-triphosphat (Fa. Sigma) in 50 mM TEA, 2 mM MgCl₂, 0.1% BSA (Fraktion V), 0.005% Brij^{®}, pH 7.5] gestartet und kontinuierlich luminometrisch vermessen. Das Maß der Stimulation durch die zu testende Substanz kann relativ zum Signal der nicht stimulierten Reaktion bestimmt werden.

Durch Zugabe von 25 µM 1*H*-1,2,4-Oxadiazolo[4,3-a]chinoxalin-1-on (ODQ) zur Enzymlösung und anschließende 30-minütige Inkubation wird die Aktivierung der Häm-freien Guanylatcyclase untersucht und mit der Stimulation des nativen Enzyms verglichen.

Repräsentative Ergebnisse zu den erfindungsgemäßen Verbindungen sind in Tabelle 2 aufgeführt:

**Tabelle 2: Aktivierende Wirkung am sGC-Enzym in vitro**

| **Beispiel Nr.** | **MEC [nM]** | **EC₅₀ [nM]** |
|---|---|---|
| 1 | 0.12 | 8.5 |
| 4 | 0.7 | 7.5 |
| 5 | 0.13 | 1.4 |
| 7 | 830 | |
| 9 | 700 | |
| 10 | 48 | 620 |
| 11 | 200 | |
| 12 | 205 | |

| | | |
|---|---|---|
| (MEC = minimale effektive Konzentration; EC₅₀ = Konzentration bei 50% der maximalen Wirksamkeit). | | |

### B-3. Gefäßrelaxierende Wirkung in vitro:

Kaninchen werden durch intravenöse Injektion von Thiopental-Natrium narkotisiert bzw. getötet (ca. 50 mg/kg) und entblutet. Die Arteria Saphena wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Repräsentative Ergebnisse zu den erfindungsgemäßen Verbindungen sind in Tabelle 3 aufgeführt:

**Tabelle 3: Gefäßrelaxierende Wirkung in vitro**

| **Beispiel Nr.** | **IC₅₀ [nM]** |
|---|---|
| 1 | 113 |
| 5 | 9140 |
| 6 | 4380 |

### B-4. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (*2*) Empfänger, die über einen Multiplexer mit einem (*3*) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer AG, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (*1*) systolischer Blutdruck (SBP), (*2*) diastolischer Blutdruck (DBP), (*3*) arterieller Mitteldruck (MAP) und (*4*) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 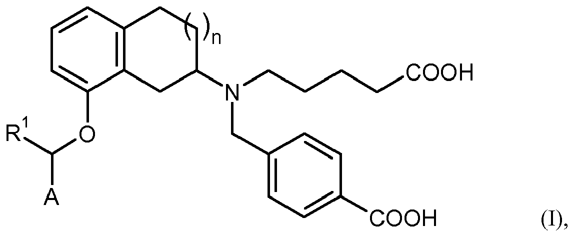 in welcher
n für die Zahl 1 steht,
R¹ für Wasserstoff oder Methyl steht,
und
A für eine Gruppe der Formel 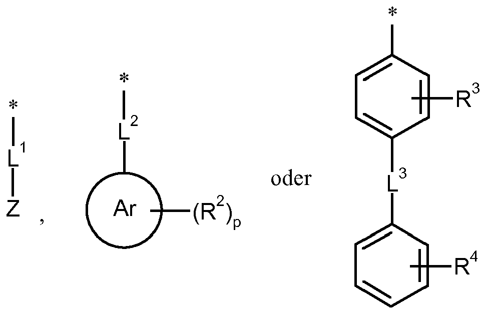 steht, worin
* die jeweilige Verknüpfungsstelle mit dem Rest des Moleküls kennzeichnet,
L¹ geradkettiges (C₁-C₅)-Alkandiyl, das ein- oder zweifach mit Methyl und ein- oder zweifach mit Fluor substituiert sein kann, bedeutet,
Z Wasserstoff, Fluor, Cyano, Trifluormethyl oder eine Gruppe der Formel 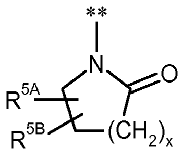 bedeutet, worin
** die Verknüpfungsstelle mit der Gruppe L¹ kennzeichnet,
x die Zahl 1, 2 oder 3 darstellt, wobei eine dieser CH₂-Gruppen gegen -O- ausgetauscht sein kann,
und
R^{5A} und R^{5B} unabhängig voneinander Wasserstoff oder Methyl darstellen,
L² eine Bindung oder geradkettiges (C₁-C₅)-Alkandiyl bedeutet,
Ar Phenyl oder 5- oder 6-gliedriges Heteroaryl mit bis zu drei Ring-Heteroatomen aus der Reihe N, O und/oder S bedeutet,
R² einen Substituenten ausgewählt aus der Reihe Fluor, Chlor, Brom, Cyano, (C₁-C₄)-Alkyl, Trifluormethyl, (C₁-C₄)-Alkoxy und Trifluormethoxy bedeutet,
p die Zahl 0, 1 oder 2 bedeutet,
wobei im Falle, dass der Substituent R² zweifach auftritt, seine individuellen Bedeutungen gleich oder verschieden sein können,
L³ eine Bindung, -O-, -CH₂-, -CH₂-CH₂- oder -CH=CH- bedeutet,
und
R³ und R⁴ unabhängig voneinander Wasserstoff oder einen Substituenten ausgewählt aus der Reihe Fluor, Chlor, Brom, Cyano, (C₁-C₄)-Alkyl, Trifluormethyl, (C₁-C₄)-Alkoxy und Trifluormethoxy bedeuten,
sowie ihre Salze, Solvate und Solvate der Salze.

2. Verbindung der Formel (I) nach Anspruch 1, in welcher
n für die Zahl 1 steht,
R¹ für Wasserstoff steht,
und
A für eine Gruppe der Formel 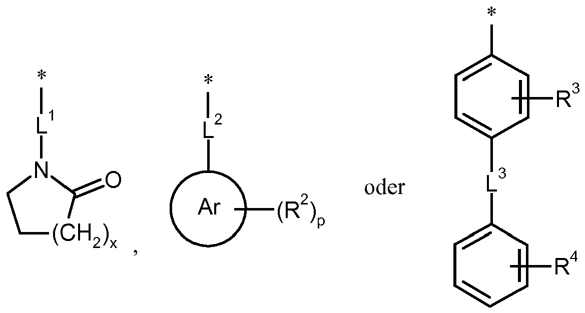 steht, worin
* die jeweilige Verknüpfungsstelle mit dem Rest des Moleküls kennzeichnet,
L¹ geradkettiges (C₂-C₄)-Alkandiyl bedeutet,
x die Zahl 1 oder 2 bedeutet, wobei eine dieser CH₂-Gruppen gegen -O- ausgetauscht sein kann,
L² eine Bindung oder -CH₂- bedeutet,
Ar Phenyl, Pyridyl, 1,2,4-Oxadiazolyl oder 1,3,4-Oxadiazolyl bedeutet,
R² einen Substituenten ausgewählt aus der Reihe Fluor, Chlor, (C₁-C₄)-Alkyl und Trifluormethyl bedeutet,
p die Zahl 0 oder 1 bedeutet,
L³ eine Bindung oder -CH₂-CH₂- bedeutet,
und
R³ und R⁴ unabhängig voneinander Wasserstoff oder einen Substituenten ausgewählt aus der Reihe Fluor, Chlor, (C₁-C₄)-Alkyl und Trifluormethyl bedeuten,
sowie ihre Salze, Solvate und Solvate der Salze.

3. Verbindung der Formel (I) nach Anspruch 1 oder 2, in welcher
n für die Zahl 1 steht,
R¹ für Wasserstoff steht,
und
A für eine Gruppe der Formel 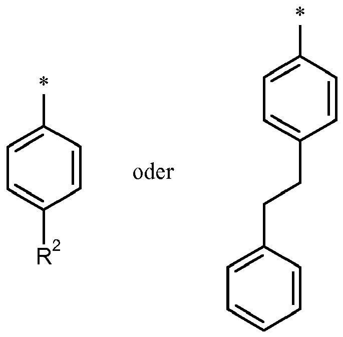 steht, worin
* die jeweilige Verknüpfungsstelle mit dem Rest des Moleküls kennzeichnet
und
R² Methyl, Ethyl, Isopropyl oder *tert*.-Butyl bedeutet,
sowie ihre Salze, Solvate und Solvate der Salze.

4. Verfahren zur Herstellung einer Verbindung der Formel (I), wie in den Ansprüchen 1 bis 3 definiert, **dadurch gekennzeichnet, dass** man eine Verbindung der Formel (II) 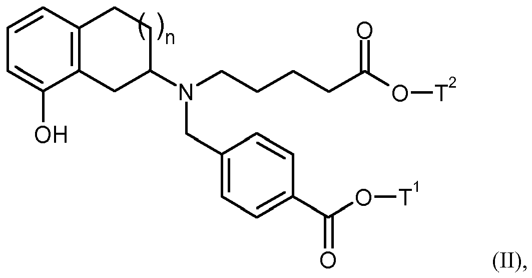 in welcher n die in den Ansprüchen 1 bis 3 angegebene Bedeutung hat
und
T¹ und T² gleich oder verschieden sind und für (C₁-C₄)-Alkyl stehen,
in Gegenwart einer Base mit einer Verbindung der Formel (III) 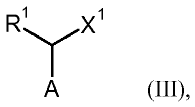 in welcher R¹ und A die in den Ansprüchen 1 bis 3 angegebenen Bedeutungen haben und
X¹ für eine Abgangsgruppe wie Chlor, Brom, Iod, Mesylat, Triflat oder Tosylat steht,
zu einer Verbindung der Formel (IV) 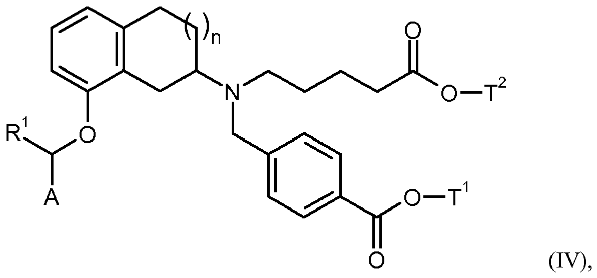 in welcher n, R¹, A, T¹ und T² jeweils die oben angegebenen Bedeutungen haben,
umsetzt und diese dann durch Hydrolyse der Ester-Gruppierungen -C(O)OT¹ und -C(O)OT² in die entsprechende Dicarbonsäure der Formel (I) überführt
und die so erhaltenen Verbindungen der Formel (I) gegebenenfalls in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

5. Verbindung, wie in einem der Ansprüche 1 bis 3 definiert, zur Verwendung bei der Behandlung und/oder Prävention von Krankheiten.

6. Verbindung zur Verwendung nach Anspruch in einem Verfahren zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

7. Verwendung einer Verbindung, wie in einem der Ansprüche 1 bis 3 definiert, zur Herstellung eines Arzneimittels zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

8. Arzneimittel enthaltend eine Verbindung, wie in einem der Ansprüche 1 bis 3 definiert, in Kombination mit einem oder mehreren inerten, nicht-toxischen, pharmazeutisch geeigneten Hilfsstoffen.

9. Arzneimittel enthaltend eine Verbindung, wie in einem der Ansprüche 1 bis 3 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

10. Arzneimittel nach Anspruch 8 oder 9 zur Verwendung bei der Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

## Claims

1. Compound of the formula (I) 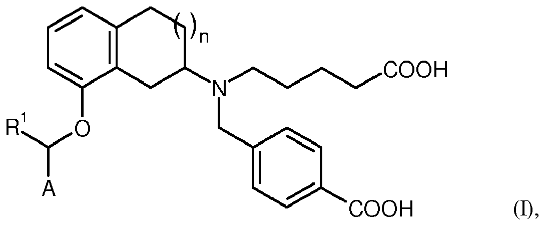 in which
n represents the number 1,
R¹ represents hydrogen or methyl
and
A represents a group of the formula 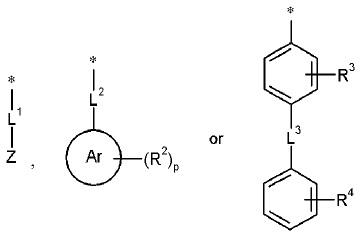 in which
* denotes the respective point of attachment to the remainder of the molecule,
L¹ represents straight-chain (C₁-C₅)-alkanediyl which may be mono- or disubstituted by methyl and mono- or disubstituted by fluorine,
Z represents hydrogen, fluorine, cyano, trifluoromethyl or a group of the formula 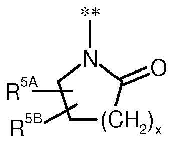 in which
** denotes the point of attachment to group L¹,
x represents the number 1, 2 or 3, where one of these CH₂ groups may be replaced by -O-,
and
R^{5A} and R^{5B} independently of one another represent hydrogen or methyl,
L² represents a bond or straight-chain (C₁-C₅)-alkanediyl,
Ar represents phenyl or 5- or 6-membered heteroaryl having up to three ring heteroatoms from the group consisting of N, O and S,
R² represents a substituent selected from the group consisting of fluorine, chlorine, bromine, cyano, (C₁-C₄)-alkyl, trifluoromethyl, (C₁-C₄)-alkoxy and trifluoromethoxy,
p represents the number 0, 1 or 2,
where, if the substituent R² occurs twice, its individual meanings may be identical or different,
L³ represents a bond, -O-, -CH₂-, -CH₂-CH₂- or -CH=CH-
and
R³ and R⁴ independently of one another represent hydrogen or a substituent selected from the group consisting of fluorine, chlorine, bromine, cyano, (C₁-C₄)-alkyl, trifluoromethyl, (C₁-C₄)-alkoxy and trifluoromethoxy,
and the salts, solvates and solvates of the salts thereof..

2. Compound of the formula (I) according to Claim 1 in which
n represents the number 1,
R¹ represents hydrogen
and
A represents a group of the formula 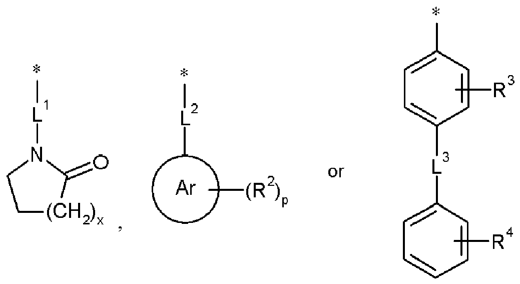 in which
* denotes the respective point of attachment to the remainder of the molecule,
L¹ represents straight-chain (C₂-C₄)-alkanediyl,
x represents the number 1 or 2, where one of these CH₂ groups may be replaced by -O-,
L² represents a bond or -CH₂-,
Ar represents phenyl, pyridyl, 1,2,4-oxadiazolyl or 1,3,4-oxadiazolyl,
R² represents a substituent selected from the group consisting of fluorine, chlorine, (C₁-C₄)-alkyl and trifluoromethyl,
p represents the number 0 or 1,
L³ represents abond or -CH₂-CH₂-
and
R³ and R⁴ independently of one another represent hydrogen or a substituent selected from the group consisting of fluroine, chlorine, (C₁-C₄)-alkyl and trifluoromethyl,
and the salts, solvates and solvates of the salts thereof..

3. Compound of the formula (I) according to Claim 1 or 2 in which
n represents the number 1,
R¹ represents hydrogen
and
A represents a group of the formula 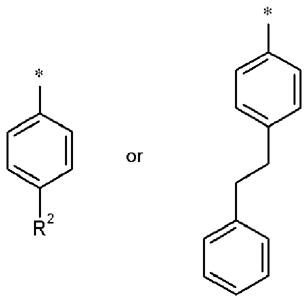 in which
* denotes the respective point of attachment to the remainder of the molecule
and
R² represents methyl, ethyl, isopropyl or *tert*-butyl,
and the salts, solvates and solvates of the salts thereof..

4. Process for preparing a compound of the formula (I) as defined in Claims 1 to 3, **characterized in that** a compound of the formula (II) 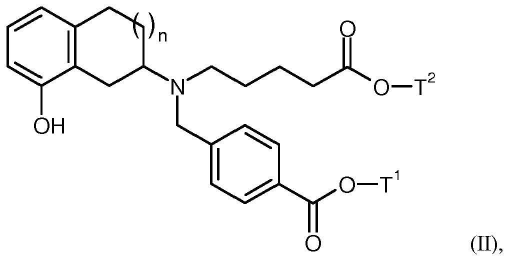 in which n has the meanings given in any of Claims 1 to 3
and
T¹ and T² are identical or different and represent (C₁-C₄)-alkyl,
is reacted in the presence of a base with a compound of the formula (III) 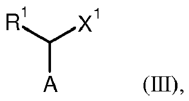 in which R¹ and A have the meanings given in any of Claims 1 to 3
and
X¹ represents a leaving group such as chlorine, bromine, iodine, mesylate, triflate or tosylate,
to give a compound of the formula (IV) 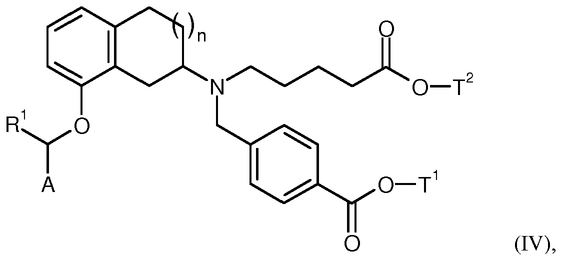 in which n, R¹, A, T¹ and T² each have the meanings given above,
and these are then converted by hydrolysis of the ester groupings -C(O)OT¹ and -C(O)OT² into the corresponding dicarboxylic acid of the formula (I)
and the compounds of the formula (I) obtained in this manner are separated where appropriate into their enantiomers and/or diastereomers, and/or where appropriate reacted with the appropriate (i) solvents and/or (ii) bases or acids to give the solvates, salts and/or solvates of the salts thereof.

5. Compound as defined in any of Claims 1 to 3 for use in the treatment and/or prevention of diseases.

6. Compound for use according to Claim 5 in a method for the treatment and/or prevention of heart failure, angina pectoris, hypertension, pulmonary hypertension, ischemias, vascular disorders, thromboembolic disorders and arteriosclerosis.

7. Use of a compound as defined in any of Claims 1 to 3 for producing a medicament for the treatment and/or prevention of heart failure, angina pectoris, hypertension, pulmonary hypertension, ischemias, vascular disorders, thromboembolic disorders and arteriosclerosis.

8. Medicament comprising a compound as defined in any of Claims 1 to 3 in combination with one or more inert, non-toxic, pharmaceutically suitable excipients.

9. Medicament comprising a compound as defined in any of Claims 1 to 3 in combination with one or more further active ingredients selected from the group consisting of organic nitrates, NO donors, cGMP-PDE inhibitors, stimulators of guanylate cyclase, agents having antithrombotic activity, agents lowering blood pressure, and agents altering lipid metabolism.

10. Medicament according to Claim 8 or 9 for use in the treatment and/or prevention of heart failure, angina pectoris, hypertension, pulmonary hypertension, ischemias, vascular disorders, thromboembolic disorders and arteriosclerosis.

## Revendications

1. Composé de formule (I) 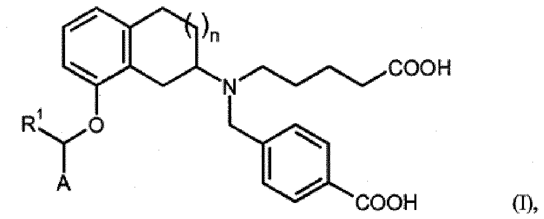 dans laquelle
n représente le nombre 1,
R¹ représente un atome d'hydrogène ou le groupe méthyle
et
A représente un groupe de formule 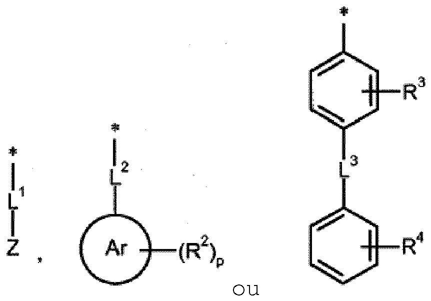 formules dans lesquelles
* désigne le point d'attachement respectif au reste de la molécule,
L¹ représente un groupe alcane(C₁-C₅)diyle à chaîne droite, qui peut être substitué une ou deux fois par méthyle et une ou deux fois par fluoro,
Z représente un atome d'hydrogène ou de fluor, le groupe cyano, trifluorométhyle ou un groupe de formule 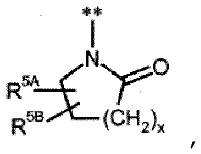 dans laquelle
** désigne le point d'attachement au groupe L¹,
x représente le nombre 1, 2 ou 3, l'un de ces groupes CH₂ pouvant être remplacé par -O-,
et
R^{5A} et R^{5B} représentent indépendamment l'un de l'autre un atome d'hydrogène ou le groupe méthyle,
L² représente une liaison ou un groupe alcane(C₁-C₅)diyle à chaîne droite,
Ar représente le groupe phényle ou un groupe hétéroaryle à 5 ou 6 chaînons, comportant jusqu'à trois hétéroatomes dans le cycle, choisis dans la série N, O et/ou S,
R² représente un substituant choisi dans la série fluoro, chloro, bromo, cyano, alkyle en C₁-C₄, trifluorométhyle, alcoxy en C₁-C₄ et trifluorométhoxy,
p représente le nombre 0, 1 ou 2,
dans le cas où le substituant R² apparaît deux fois, ses significations individuelles pouvant être identiques ou différentes,
L³ représente une liaison, -O-, -CH₂-, -CH₂-CH₂- ou -CH=CH-,
et
R³ et R⁴ représentent indépendamment l'un de l'autre un atome d'hydrogène ou un substituant choisi dans la série fluoro, chloro, bromo, cyano, alkyle en C₁-C₄, trifluorométhyle, alcoxy en C₁-C₄ et trifluorométhoxy,
ainsi que ses sels, produits de solvatation et produits de solvatation des sels.

2. Composé de formule (I) selon la revendication 1, dans lequel
n représente le nombre 1,
R¹ représente un atome d'hydrogène,
et
A représente un groupe de formule 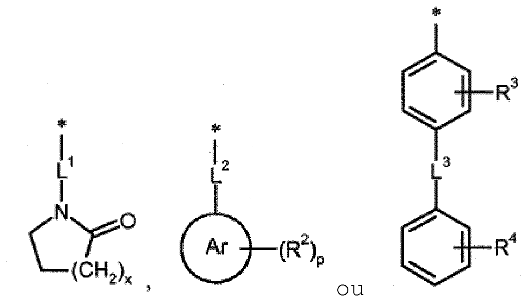 formules dans lesquelles
* désigne le point d'attachement respectif au reste de la molécule,
L¹ représente un groupe alcane(C₂-C₄)diyle à chaîne droite,
x représente le nombre 1 ou 2, l'un de ces groupes CH₂ pouvant être remplacé par -O-,
L² représente une liaison ou -CH₂-,
Ar représente le groupe phényle, pyridyle, 1,2,4-oxadiazolyle ou 1,3,4-oxadiazolyle,
R² représente un substituant choisi dans la série fluoro, chloro, alkyle en C₁-C₄ et trifluorométhyle,
p représente le nombre 0 ou 1,
L³ représente une liaison ou -CH₂-CH₂-,
et
R³ et R⁴ représentent indépendamment l'un de l'autre un atome d'hydrogène ou un substituant choisi dans la série fluoro, chloro, alkyle en C₁-C₄ et trifluorométhyle,
ainsi que ses sels, produits de solvatation et produits de solvatation des sels.

3. Composé de formule (I) selon la revendication 1 ou 2, dans lequel
n représente le nombre 1,
R¹ représente un atome d'hydrogène,
et
A représente un groupe de formule 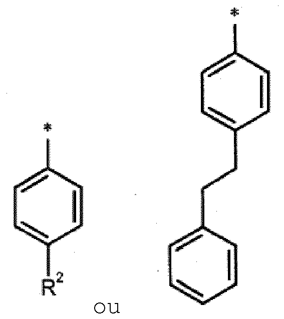 formules dans lesquelles
* désigne le point d'attachement respectif au reste de la molécule,
et
R² représente le groupe méthyle, éthyle, isopropyle ou *tert*-butyle
ainsi que ses sels, produits de solvatation et produits de solvatation des sels.

4. Procédé pour la préparation d'un composé de formule (I), tel que défini dans les revendications 1 à 3, **caractérisé en ce qu'**on fait réagir un composé de formule (II) 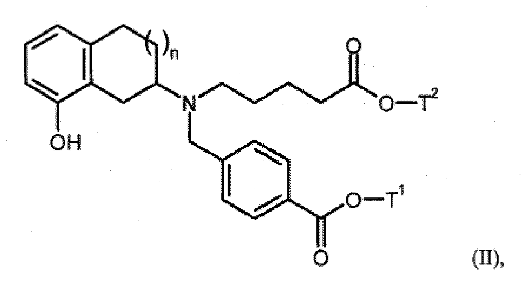 dans laquelle n a les significations indiquées dans les revendications 1 à 3
et
T¹ et T² sont identiques ou différents et représentent un groupe alkyle en C₁-C₄,
en présence d'une base, avec un composé de formule (III) 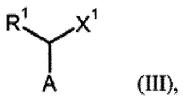 dans laquelle R¹ et A ont les significations indiquées dans les revendications 1 à 3
et
X¹ représente un groupe partant tel que chloro, bromo, iodo, mésylate, triflate ou tosylate,
pour aboutir à un composé de formule (IV) 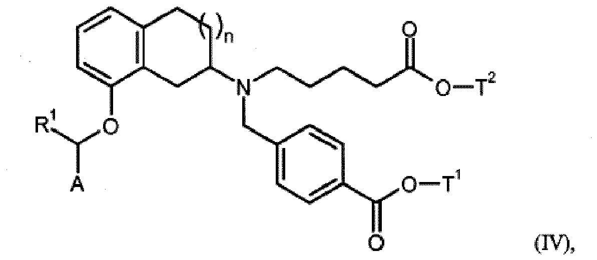 dans laquelle n, R¹, A, T¹ et T² ont chacun les significations indiquées plus haut,
et on convertit ensuite ce dernier, par hydrolyse des groupements ester -C(O)OT¹ et -C(O)OT², en l'acide dicarboxylique correspondant de formule (I)
et éventuellement on fractionne les composés de formule (I) ainsi obtenus en leurs énantiomères et/ou diastéréoisomères et/ou éventuellement on les convertit, à l'aide (i) des solvants correspondants et/ou (ii) des bases correspondantes ou des acides correspondants, en leurs produits de solvatation, sels et/ou produits de solvatation des sels.

5. Composé, tel que défini dans l'une quelconque des revendications 1 à 3, pour l'utilisation dans le traitement et/ou la prévention de maladies.

6. Composé pour l'utilisation selon la revendication 5, dans un procédé pour le traitement et/ou la prévention de l'insuffisance cardiaque, de l'angine de poitrine, de l'hypertonie, de l'hypertonie pulmonaire, d'ischémies, de maladies vasculaires, de maladies thromboemboliques et de l'artériosclérose.

7. Utilisation d'un composé, tel que défini dans l'une quelconque des revendications 1 à 3, pour la fabrication d'un médicament destiné au traitement et/ou à la prévention de l'insuffisance cardiaque, de l'angine de poitrine, de l'hypertonie, de l'hypertonie pulmonaire, d'ischémies, de maladies vasculaires, de maladies thromboemboliques et de l'artériosclérose.

8. Médicament contenant un composé, tel que défini dans l'une quelconque des revendications 1 à 3, en association avec un ou plusieurs adjuvants inertes, non toxiques, pharmaceutiquement convenables.

9. Médicament contenant un composé, tel que défini dans l'une quelconque des revendications 1 à 3, en association avec une ou plusieurs autres substances actives choisies dans le groupe constitué par des nitrates organiques, des donneurs de NO, des inhibiteurs de cGMP-PDE, des stimulants de la guanylate-cyclase, des agents à action antithrombotique, des agents abaissant la tension artérielle ainsi que des agents modifiant le métabolisme lipidique.

10. Médicament selon la revendication 8 ou 9, destiné à l'utilisation dans le traitement et/ou la prévention de l'insuffisance cardiaque, de l'angine de poitrine, de l'hypertonie, de l'hypertonie pulmonaire, d'ischémies, de maladies vasculaires, de maladies thromboemboliques et de l'artériosclérose.
